# EUROPEAN PATENT APPLICATION

(11) **EP 1 234 577 A1**
(43) Date of publication of application: **28.08.2002**
(21) Application number: 00978007.3
(22) Date of filing: 29.11.2000
(51) Int. Cl.: A61K 31/765, A61P 43/00

(54) **RADIOPROTECTING AGENT**

(30) Priority: 03.12.1999 JP 34404399
(71) Applicant: AMATO PHARMACEUTICAL PRODUCTS, LTD., Fukuchiyama-shi, Kyoto 620-0932 (JP)
(72) Inventor: MURAYAMA, Chieko, Ebina-shi, Kanagawa 243-0431 (JP); OIZUMI, Yukio, Isehara-shi, Kanagawa 259-1117 (JP); MURAKAMI, Masahiro, Fukuchiyama-shi, Kyoto 620-0055 (JP)
(74) Representative: Sternagel, Fleischer, Godemeyer & Partner Patentanwälte
(86) International application number: JP0008423
(87) International publication number: WO01039782

(57) **Abstract**

The object of the present invention is to provide a radiation preventing agent and food and drink using the same, that have a function of giving protection against radiation and have no harmful side effects. The present invention provides a radiation protecting agent which comprises, as an active ingredient, a mixture of cyclic and/or straight chain poly lactic acids having a condensation degree of 3 to 19.

## Description

### TECHNICAL FIELD

The present invention relates to a radiation protecting agent which has a protecting effect against radiation disorder in an organism. More specifically, the present invention relates to a radiation protecting agent which comprises, as an active ingredient, a mixture of poly lactic acids having a condensation degree of certain range.

### BACKGROUND ART

Workers in atomic power plants, X-ray technicians who use X-rays, and technicians and doctors engaged in radiation therapy for cancer and the like, do their works while avoiding exposure to radiation. However, it is difficult to completely avoid exposure, and if they do their works for a long time, there is possibility of being exposed to a large amount of radiation. Further, if persons are subjected to whole-body exposure to radiation at one time by accident of atomic power plants or the like, these persons die or express radiation disorders at the organ/tissue level and cell level such as genital function disorder, bone marrow function disorder or skin disorder after a certain latency period. For the purpose of preventing disorders in the body due to radiation, exposure of an individual to radiation is regulated by laws and regulations, or internal disorder prevention rules of establishments which deal with radiation. However, even with these radiation protection standards, it is impossible to completely prevent disorders due to radiation exposure. Further, since there is a latent period from radiation exposure to appearance of the above described disorders, one can first know exposure by appearance of symptoms . However, treatment of such symptoms is very difficult in the current medical standard.

Further, cancer is the cause of death for approximately one quarter of Japanese, and it is expected that the number of cancer patients increases in the future. Major methods of treating cancer include surgical operation, radiation therapy and chemotherapy, and combinations of these therapies are performed at present.

In cancer patients who receive radiation therapy, a substantial amount of radiation is irradiated at one time on the malignant tumor area to be treated, but normal tissue in the vicinity of the malignant tumor is also irradiated. Consequently, in such exposed normal tissue, free radicals generated in the body by radiation cause an oxidative chemical reaction of intracellular molecules such as those in the cell membrane and chromosomes, and this triggers a serious disorder such as cessation of cell proliferation (cell death) and induction of mutation.

Radiation therapy is, along with surgical operation, a radical treatment of cancer. However, since patients exposed to radiation exhibit various disorders, the utility of radiation therapy is lowered. Thus, if there could be developed an agent for the prevention and treatment of radiation disorder that can give protection against various disorders arising from irradiation, then it would be possible to dramatically increase the amount of radiation used in radiation therapy, and increase the efficacy of radiation therapy.

As agents that give protection against radiation disorder, various types of amino thiol which are hydrogen radical donors, such as β-mercaptoethylamine, have long been known (Sugawara, T. et al., Radiation and Medicine, Kyoritu, 1986). However, the above-described agents, due to their strong side effects, cannot be administered in an amount necessary to exhibit an effective protective effect, and thus are not in practical use. On the other hand, α-tocopherol (a representative vitamin E) is a compound having a function of donating a hydrogen atom from the hydroxyl group at position 6 on the chroman ring and eliminating a free radical, and is well known as an antioxidant. However, vitamin E is a viscous oily substance that does not dissolve in water, since it has a long carbohydrate chain (phytyl group) in its molecule. For this reason, where vitamin E is administered for the purpose of preventing damage of an organism due to free radicals, there is a problem that it cannot be used in a form of solution such as oral medicine or injection agent.

Therefore, the development of a novel radiation protecting agent that has a function of protecting an organism from radiation exposure is strongly desired.

### DISCLOSURE OF THE INVENTION

The object of the present invention is to provide a radiation preventing agent and food and drink using the same, that have a function of giving protection against radiation and have no harmful side effects.

As a result of intensive studies to solve the above problem, the present inventors found that a mouse could be protected from radiation by administering a mixture of cyclic and/or straight chain poly lactic acids having a condensation degree of 3 to 19 to the mouse prior to irradiation with radiation, thereby completing the present invention.

From studies that have been made so far, it has been reported that a mixture of cyclic and/or straight chain poly L-lactic acids having a condensation degree of 3 to 19 is useful as an antineoplastic agent (Japanese Patent Application Laying-Open (Kokai) Nos. 9-227388 and 10-130153) and also as a QOL improving agent for cancer patients (Japanese Patent Application No. 11-39894 Specification; Bulletin of Japan Society of Clinical Oncology, Vol. 33, No. 3, p. 493). Furthermore, it has also been found that the mixture of cyclic and/or straight chain poly lactic acids having a condensation degree of 3 to 19 has a hypoglycemic action and is useful as a medicament for prevention and/or treatment of diabetes or diabetes complications (Japanese Patent Application No. 11-224883) and is also useful for suppressing an appetite, promoting a basal metabolism, and improving and/or preventing obesity (Japanese Patent Application No. 11-280931), but it has been found for the first time by the present inventors that this mixture of poly lactic acids is useful for giving protection against radiation.

Thus, according to the first aspect of the present invention, there is provided a radiation protecting agent which comprises, as an active ingredient, a mixture of cyclic and/or straight chain poly lactic acids having a condensation degree of 3 to 19.

The radiation protecting agent of the present invention is used, for example, for prevention and/or therapy of disorders due to radiation therapy.

Preferably, the lactic acid that is a repeating unit in the poly lactic acid consists substantially of L-lactic acid.

Preferably, the mixture of cyclic and/or straight chain poly lactic acids having a condensation degree of 3 to 19 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

Preferably, condensation by dehydration is performed by stepwise decompression and temperature rise under nitrogen gas atmosphere.

Preferably, reverse phase column chromatography is performed by ODS column chromatography.

According to another aspect of the present invention, there are provided food and drink for the protection against radiation , which comprises, as an active ingredient, a mixture of cyclic and/or straight chain poly lactic acids having a condensation degree of 3 to 19. The preferred embodiments of the mixture of poly lactic acids used in the food and drink of the present invention are as mentioned above.

According to still another aspect of the present invention, there is provided the use of a mixture of cyclic and/or straight chain poly lactic acids having a condensation degree of 3 to 19 in the production of a radiation protecting agent and food and drink for protection against radiation.

According to a still further aspect of the present invention, there is provided a method for giving protection against radiation, which comprises a step of administering an effective amount of a mixture of cyclic and/or straight chain poly lactic acids having a condensation degree of 3 to 19 to mammals such as humans.

### BRIEF DESCRIPTION OF THE DRAWINGS

Figure 1 shows a mass spectrum of the mixture of poly lactic acids obtained by Production Example 1 of the present specification.
Fig. 2 is a graph showing a surviving rate of C3H/HeN mice (control group) after whole-body irradiation.
Fig. 3 is a graph showing a surviving rate of C3H/HeN mice (CPL-administered group) after whole-body irradiation.
Fig 4. is a graph showing a mortality rate of C3H/HeN mice at 30th day after whole-body irradiation. White circles show control group, and black circles show CPL-administered group.

### THE BEST MODE FOR CARRYING OUT THE INVENTION

A "radiation protecting agent" used in the present specification refers to an agent for protecting a cell or organism from adverse effects which are given to cells by radiation exposure. The radiation protecting agent of the present invention is used to protect cells from harmful cell disorders arising from radiation exposure. Radiation is high-energy radiation such as X-rays and gamma rays which have an action of producing ion pairs within substances. Radiation exposure can occur as a result of a radioactive environment arising from nuclear explosion, leak of radioactive material, accession to radioactive material and the like, and can also occur as a result of radiation therapy such as radiation therapy for various types of tumors.

Examples of the above-mentioned adverse influence on a cell include damage of cell DNA such as DNA chain cleavage, disorganization of cell function, cell death, and induction of tumor. Radiation disorders are divided into acute disorders which occur in tissues where cell rejuvenation constantly occurs (mucosa of the skin, oral cavity, small and large intestine, vagina, etc.) , and chronic disorders which occur in tissues where cell rejuvenation is slow or does not take place at all. Chronic disorders further include physical disorders and genetic disorders. Examples of radiation disorders include, but are not limited to, radiation gastritis, radiation necrosis, radiation ulceration, radiation burns, radiation hepatitis, radiation stomatitis, radiation osteonecrosis, radiation syndrome (radiation sickness), radiation epithelium, radiation esophagitis, radiation nephritis, radiation myelopathy, radiation fibrosis, radiation proctosis, radiation mucitis, radiation pneumonia, radiation cataract, radiation keratitis, radiation leukopenia, radiation dermatitis, radiation anemia, radiation cystitis, and radiation leukemia, and the like. The radiation protecting agent of the present invention can be used for the treatment and prevention of such disorders caused by radiation exposure.

The radiation protecting agent of the present invention can be used as a coadjuvant for radiation therapy in order to prevent and/or treat disorders due to radiation therapy. For example, radiation exposure in radiation therapy for the treatment of tumor can kill cancer cells. However, radiation also brings about harmful cell effects against normal cells. The agent of the present invention can protect cells against such harmful cell effects by preventing or eliminating these harmful effects of radiation or reducing their level.

A "radiation therapy" in the present specification refers to, for example, radiation therapy for a tumor. Herein, tumor includes, but is not limited to, luekemias such as acute lymphoblastic, acute myelogenic, chronic lymphocytic, acute myoblstic and chronic myoblastic leukemia; cancers of the neck, esophagis, stomach, pancreas, breast, ovaries, small intestine, colon and lung, etc.; sarcomas such as osteosarcoma, lipoma, liposarcoma, angioma and angiosarcoma, etc.; non-melatonic and melatonic melanoma; and mixed tumors such as carcinosarcoma, lymphoid tissue type, endoplasmic reticulum, cytosarcoma, Hodgkin's disease, and non-Hodgkins lymphoma.

In the radiation protecting agent of the present invention, a mixture of cyclic and/or straight chain poly lactic acids having a condensation degree of 3 to 19 is used as an active ingredient.

The term "a mixture of poly lactic acids" used in the present invention means a mixture wherein cyclic and/or straight chain poly lactic acids having a condensation degree of 3 to 19 are present at any ratio. That is to say, the term "mixture" does not only mean a mixture of poly lactic acids having any condensation degree ranging from 3 to 19, but is also used as a concept including a mixture of cyclic and straight chain poly lactic acids. As is described below in the present specification, "a mixture of poly lactic acids" can be obtained by condensing lactic acids by dehydration and then performing purification by a suitable method. Although the term "a mixture of poly lactic acids" is used in the present specification for the sake of convenience, this term also includes a poly lactic acid consisting of a single ingredient such as a cyclic poly lactic acid having single condensation degree or a straight chain poly lactic acid having single condensation degree.

The term "condensation degree" is used to mean the number of lactic acid unit that is a repeating unit in poly lactic acids. For example, the cyclic poly lactic acid is assumed to have the following structural formula wherein n represents condensation degree (n = 3 to 19).

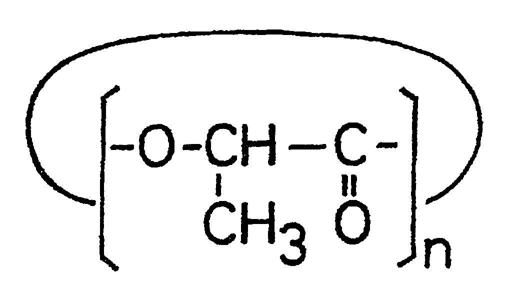

When "lactic acid" is simply referred to in the present specification, this lactic acid includes all of L-lactic acid, D-lactic acid or a mixture comprising these types of lactic acid at any ratio. Preferably in the present invention, the lactic acid consists substantially of L-lactic acid. The term "substantially" is used herein to mean that the ratio of L-lactic acid units in a mixture of poly lactic acids (number of L-lactic acid unit / number of L-lactic acid unit + number of D-lactic acid unit × 100) is, for example, 70% or more, preferably 80% or more, more preferably 85% or more, further more preferably 90% or more, and particularly preferably 95% or more. The ratio of L-lactic acid units in a mixture of poly lactic acids depends on the ratio of L-lactic acid and D-lactic acid that exist in lactic acids used as a starting substance.

The methods for producing a mixture of cyclic and/or straight chain poly lactic acids having a condensation degree of 3 to 19 are not particularly limited, and the mixture of poly lactic acids can be obtained by the production methods described, for example, in Japanese Patent Application Laying-Open (Kokai) Nos. 9-227388 and 10-130153 or Japanese Patent Application No. 11-39894 (All publications cited herein are incorporated herein by reference in their entirety).

More specifically, for example, a mixture of cyclic and/or straight chain poly lactic acids having a condensation degree of 3 to 19 can be obtained by the following method A.

### Method A:

First, lactic acid (preferably, lactic acid substantially consisting of L-lactic acid) is condensed by dehydration under an inactive atmosphere. Examples of the inactive atmosphere include nitrogen gas and argon gas, and nitrogen gas is preferred.

Dehydration and condensation reaction is carried out at a temperature of 110°C to 210°C, preferably 130°C to 190°C under normal pressure to reduced pressure of approximately 1mmHg, and particularly preferably the reaction is carried out by stepwise decompression and stepwise temperature rise. A reaction period can be determined as appropriate. For example, the reaction can be carried out for 1 to 20 hours. Where stepwise decompression and stepwise temperature rise are applied, reaction is performed by dividing the reaction period into two or more partial reaction periods, and then determining pressure and temperature for each of the reaction periods . Where stepwise decompression is applied, pressure can be reduced, for example, from a normal pressure to 150mmHg and then to 3mmHg. Where stepwise temperature rise is applied, temperature can be raised, for example, from 145°C to 155°C and then to 185°C. Practically, the reaction can be carried out by using these conditions in combination, for example, 145°C, normalpressure, 3hours; 145°C, 150mmHg, 3 hours; 155°C, 3mmHg, 3 hours; and 185°C, 3mmHg, 1.5 hours.

Subsequently, ethanol and methanol are added to the reaction mixture obtained by the dehydration and condensation reaction, and the mixture is filtered. The obtained filtrate is dried to obtain ethanol- and methanol-soluble fractions. The term "ethanol- and methanol-soluble fractions" is used in the present specification to mean fractions soluble in a mixed solution of ethanol and methanol. In order to obtain ethanol and methanol-soluble fractions, a reaction mixture obtained by dehydration and condensation reaction is mixed with ethanol and methanol, where the ratio of ethanol and methanol can be determined as appropriate. For example, the ratio is ethanol:methanol = 1 : 9. The order, method and the like for adding ethanol and methanol to a reaction mixture are not limited, and may be selected as appropriate. For example, ethanol may be added at first to the reaction mixture obtained by the dehydration and condensation reaction, and then methanol may be added thereto.

The thus obtained ethanol- and methanol-soluble fractions are subjected to reverse phase column chromatography, especially to chromatography where an octadecylsilane (ODS) column is used. First, fractions eluted with 25 to 50 weight % acetonitrile aqueous solution of pH 2 to 3 are removed, and then fractions eluted with 90 weight % or more acetonitrile aqueous solution of pH 2 to 3, preferably 99 weight % or more acetonitrile aqueous solution, are collected so as to obtain a mixture of cyclic and/or straight chain poly lactic acids having a condensation degree of 3 to 20.

The thus obtained mixture of cyclic and/or straight chain poly lactic acids is neutralized with an alkaline substance such as sodium hydroxide, and is dried under reduced pressure, and then according to standard techniques, the mixture can be formulated in a desired form as mentioned below.

Other examples of the methods for producing a mixture of cyclic and/or straight chain poly lactic acids having a condensation degree of 3 to 19 used in the present invention include a method described in Japanese Patent Application No. 11-265715 (hereinafter referred to as method B), or a method described in Japanese Patent Application No. 11-265732 (hereinafter referred to as method C) (All publications cited herein are incorporated herein by reference in their entirety) . Methods B and C will be described specifically below.

### Method B:

Method B is a method for producing a cyclic lactic acid oligomer which comprises polymerizing lactid (3,6-dimethyl-1,4-dioxane-2,5-dione) in the presence of an alkali metal compound represented by RYMe [wherein R represents an aliphatic group, aromatic group, substituted or unsubstituted silyl group, or lactamide group (-CH(CH₃)CONH₂ group), Y represents oxygen atom, sulfur atom, or NR', in which R' represents hydrogen atom, aliphatic group or aromatic group, and Me represents alkali metal].

An aliphatic carbohydrate group in the present specification may be straight chain, branched-chain, cyclic, or any combination of these, and may be a saturated or unsaturated group. The carbon number of the aliphatic carbohydrate group is 1 to 12, preferably 1 to 6. Examples of the aliphatic carbohydrate group include a chain (including both straight chain and branched-chain) alkyl group such as methyl, ethyl, propyl, butyl, octyl and dodecyl; and a cycloalkyl group (for example, cyclopropyl, cyclobutyl, cyclopentyl, and cyclohexyl).

An aromatic carbohydrate group in the present specification includes an aryl group which may have a substituted group such as an alkyl group, and an arylalkyl group, and the carbon number thereof is 6 to 12, preferably 6 to 10. Examples of the aryl group which may have a substituted group such as an alkyl group include phenyl, tolyl, and naphthyl. Examples of the arylalkyl group include benzyl, phenethyl, and naphthylmethyl.

Examples of a substituted group of a substituted or unsubstituted silyl group include an aliphatic carbohydrate group or aromatic carbohydrate group. Specific examples of the substituted silyl group include a trimethylsilyl group, triphenylsilyl group or t-butyldimethylsilyl group.

Examples of alkali metal represented by Me include lithium, sodium or potassium, and lithium is preferred.

An alkali metal compound represented by RYMe can be obtained by allowing alkyl-alkalimetal, such as n-butyl lithium, to react with R'-YH (wherein R' represents an aliphatic carbohydrate group or aromatic carbohydrate group, and Y represents oxygen atom or sulfur atom).

Specifically, reaction can be carried out by preparing a solution by dissolving an alcohol compound or thiol compound represented by R'-YH in an appropriate solvent (for example, an ether-based solvent, such as anhydrous tetrahydrofuran or anhydrous diethyl ether), adding alkyl-alkali metal, such as n-butyl lithium, to the solution in an amount almost equivalent to that of the alcohol compound or thiol compound, and then stirring the solution.

Reaction may be carried out at a low temperature (for example, -78°C) for several minutes to 1 hour.

When a cyclic lactic acid oligomer used in the present invention is produced by allowing lactid (3,6-dimethyl-1,4-dioxane-2,5-dione) to react in the presence of an alkali metal compound (RYMe), the cyclic lactic acid oligomer can be produced by adding a lactid solution in an appropriate solvent (for example, anhydrous tetrahydrofuran) to a reaction mixture containing the alkali metal compound obtained as described above, and stirring the solution.

The molar ratio of the amounts of the alkali metal compound (RYMe) and lactid is 1:1 to 1:10, preferably about 1:2 to 1:5, for example, 1:3 or 1:4.

A reaction temperature range is -78°C to room temperature. Reaction is preferably carried out by starting from a temperature of -78°C, and gradually raising it to room temperature. In addition, a reaction pressure is not specifically limited, and normal pressure is preferred.

As described above, the reaction is preferably carried out in the presence of a solvent. A preferred reaction solvent is a solvent inactive in reaction. For example, an ether-based solvent (anhydrous tetrahydrofuran, anhydrous diethylether, or the like) can be used.

Reaction is preferably performed under an inactive gas atmosphere such as nitrogen gas and argon gas.

The composition (that is, the mixing ratio of a cyclic lactic acid oligomer and a chain lactic acid oligomer) of the lactic acid oligomer obtained as described above fluctuates depending on the alkali metal compound used as a reaction assistant. Where an alkali metal compound of alkyl alcohol having a carbon number of 1 to 3 (ROMe) (wherein R represents an alkyl group with carbon number 1 to 3, and Me represents alkali metal) is used as an alkali metal compound, a mixture of a cyclic lactic acid oligomer and a chain oligomer (proportion of the cyclic lactic acid oligomer: 80 to 85 weight %) is obtained. When a lithium compound of alkyl alcohol having a carbon number of 4 or more such as t-butyl alcohol, or thiophenol compound is used as an alkali metal compound, substantially only a cyclic lactic acid oligomer can be selectively obtained.

The polymerization degree of the cyclic lactic acid oligomer used in the present invention is 3 to 19, preferably 3 to 17. This polymerization degree fluctuates depending on the type of alkali metal compound to be used, reaction temperature and reaction period.

Further, a mixture of cyclic (and chain, depending on the case) lactic acid oligomers with different polymerization degrees is thought to be present in the reaction product resulting from polymerization reaction of lactid in the presence of the above alkali metal compound. In the present invention, a mixture comprising lactic acid oligomers with different polymerization degrees can be used. However, a single lactic acid oligomer having a single polymerization degree may be obtained by purifying with a technique (for example, gel filtration, HPLC or the like) appropriate for separating compounds of differing molecular weights from a reaction mixture containing the above lactic acid oligomers with different polymerization degrees, and may be used herein.

In the above method of producing a cyclic lactic acid oligomer, substantially only cyclic lactic acid oligomers can also be selectively obtained by carrying out reaction in the same manner as described above with the exception that an alkali metal compound of lactamide (in particular, a lithium compound) (that is, a compound wherein R represents -CH(CH₃)CONH₂ group) is used as an alkali metal compound.

### Method C:

This method comprises:
(i) a first heating step which comprises heating lactic acid under a pressure condition of 350 to 400 mmHg and to a temperature of 120 to 140°C so as to perform dehydration and condensation, and distilling off and removing only by-product water without distilling lactid off;
(ii) a second heating step for synthesizing a product condensed by dehydration comprising chain lactic acid oligomers as the main ingredient, which comprises, after completion of the first heating step, heating the reaction product to a temperature of 150 to 160°C while reducing the reaction pressure to 15 to 20 mmHg at a decompression rate of 0.5 to 1 mmHg/min, wherein only by-product water is distilled off and removed while avoiding distillation of lactid; and after the reaction pressure is reduced to 15 to 20 mmHg, maintaining the reaction under the same pressure condition and at a reaction temperature of 150 to 160°C;
(iii) a third heating step for synthesizing cyclic oligomers which comprises, after completion of the second heating step, heating under a pressure condition of 0.1 to 3 mmHg and at 150 to 160°C to cyclize the chain lactic oligomer.

In this method, first, in the first heating step, lactic acid is heated under reduced pressure to perform dehydration and compression reaction. In this case the reaction period is 3 to 12 hours, preferably 5 to 6 hours. To allow the reaction in the first heating step to proceed smoothly, by-product water produced by condensation of lactic acids by dehydration is distilled off. At this time, distillation of by-product water is performed such that lactid, which is the dehydrated condensed product of two molecules of lactic acid, is not distilled off. To achieve such purpose, the reaction pressure is maintained at a reduced pressure, preferably 300 to 500 mmHg, more preferably 350 to 400 mmHg. Under this pressure condition, heating is performed at a temperature range of 100 to 140°C, preferably 130 to 140°C. The reaction product produced by reaction in the first heating step mainly comprises as the main ingredient a dehydrated condensed product of 3 to 23 molecules of lactic acid.

To obtain oligomers having an increased average degree of polymerization in the second heating step after completion of the above first heating step, heating is performed at a temperature higher than the reaction temperature of the above first heating step, preferably at 145°C to 180°C, more preferably 150°C to 160°C, while the reaction pressure is reduced to 10 to 50 mmHg, preferably 15 to 20 mmHg, so that dehydration and condensation reaction is further continued.

As with the reaction in the above first heating step, reaction is performed under a condition where by-product water, but not lactid, is distilled off, to allow the reaction to proceed smoothly. The rate at which reaction pressure is reduced to a pressure in the above range (decompression rate) is normally required to be maintained within a range of 0.25 to 5 mmHg/min, preferably 0.5 to 1 mmHg/min, in order to avoid distillation of lactid and increase the reaction efficiency. A decompression rate lower than the above range is not preferred because it will increase the time required to reduce pressure to a given pressure. On the other hand, a decompression rate higher than the above range is also not preferred because it will cause lactid to be distilled off together with by-product water.

After the reaction pressure is reduced to a certain pressure, reaction is further continued at that reactionpressure. The heating time period in this case is 3 to 12 hours, preferably 5 to 6 hours.

A lactic acid oligomer having an average polymerization degree of 3 to 30, preferably 3 to 23 is obtained by the reaction in the above second heating step. The proportion of cyclic oligomers in the oligomers in this case is normally about 70 to 80 weight %.

In the third heating step, after completion of the above second heating step, a reaction pressure is maintained at 0.25 to 5 mmHg, preferably 0.5 to 1 mmHg, and reaction is further continued at a temperature of 145 to 180°C, preferably 150 to 160°C. A reaction period is 3 to 12 hours, preferably 5 to 6 hours. By-product water produced in this case is also distilled off. In this case, distillation of lactid is preferably avoided. However, since the reaction product contains almost no lactid, it is not required to specially lower the decompression rate.

Lactic acid oligomers produced by reaction in the above third heating step have an average polymerization degree of 3 to 30, preferably 3 to 23, and contain cyclic oligomer in the proportion of 90 weight % or more, preferably 99 weight % or more.

The above methods A, B and C merely show some of specific examples of methods of producing a mixture of poly lactic acids used in the present invention. A mixture of poly lactic acids which is produced by other methods can also be used in the present invention.

The dosage form of the radiation protecting agent of the present invention is not particularly limited, and any form suitable for the purpose of therapy or prevention can be selected from dosage forms for oral or parenteral administration. Examples of dosage forms suitable for oral administration include a tablet, a capsule, a powder, a granule, a parvule, a syrup, a solution, an emulsion, a suspension, a chewable tablet, and the like. Examples of dosage forms suitable for parenteral administration include, but are not limited to, transcutaneous absorbing agents in the form of an injection (e.g. a subcutaneous, intramuscular or intravenous injection, and the like) , a drop, an inhalant, a nebula, a suppository, a gel, ointment or the like, and transcutaneous absorbing agents in the form of a trans-mucous absorbing agent, a patch agent, a tape agent or the like. Dosage forms for oral administration is preferred.

Liquid formulations suitable for oral administration such as a solution, emulsion or syrup can be produced using water; sugars such as sucrose, sorbit or fructose; glycols such as polyethylene glycol or propylene glycol; oils such as sesame oil, olive oil or soybean oil; antiseptics such as p-hydroxybenzoate; and flavors such as strawberry flavor and peppermint. In order to produce solid formulations such as capsule, tablet, powder or granule, there can be used an excipient such as lactose, glucose, sucrose or mannite; a disintegrator such as starch or sodium alginate; a lubricant such as magnesium stearate or talc; a binder such as polyvinyl alcohol, hydroxypropylcellulose or gelatin; a surfactant such as fatty acid ester; and a plasticizer such as glycerine, and the like.

Formulations for an injection or drop that are suitable for parenteral administration preferably comprise, as an active ingredient, the above substance in a dissolved or suspended state in a sterilized aqueous medium which is isotonic to the recipient's blood. For example, in the case of an injection, a solution can be prepared using an aqueous medium consisting of a saline solution, a glucose solution or a mixture of a saline solution and a glucose solution. In the case of a formulation for intestinal administration, it can be prepared using carriers such as theobroma oil, hydrogenated lipids or hydrogenated carboxylic acid, and can be provided as a suppository. In order to produce a nebula, the above substance as an active ingredient may be dispersed as microparticles, and a carrier which does not irritate the recipient's cavitas oris and respiratory tract mucosa and which facilitates absorption of the active ingredient can be used. Specific examples of carriers include lactose, glycerine, and the like. Formulations having a form such as aerosol or dry powder may be prepared depending on the properties of the substance of an active ingredient and the carrier to be used. One or two or more auxiliary ingredients selected from glycols, oils, flavors, an antiseptic, an excipient, a disintegrator, alubricant, abinder, asurfactant, aplasticizer and the like may be added to these formulations for parenteral administration.

The dose and dosage frequency of the radiation protecting agent of the present invention are determined as appropriate, depending on various factors such as type and irradiation dosage of radiation to be protected, type and severity of the disease to be treated, dosage form, and condition such as age or body weight of a patient. Generally, the dose of an active ingredient per day is 20 to 2,000 mg/kg, preferably 20 to 200 m/kg, and more preferably 50 to 150 mg/kg. It is preferred that the above dose of the medicament is dividedly applied about once to 4 times per day, preferably about twice to 4 times per day.

Where the radiation protecting agent of the present invention is used for radiation protection, it is preferred that the agent is administered prior to irradiation with radiation (exposure). For example, the agent can be administered from 2 months to 2 weeks prior to irradiation with radiation (exposure).

The radiation protecting agent of the present invention can be administered to any mammal including humans, and is preferably administered to human.

The mixture of cyclic and/or straight chain poly lactic acids having a condensation degree of 3 to 19 used in the present invention is used not only as a medicament, but also may be mixed into a drinkable preparation, such as a nutrition supplement drinkable preparation, or can be mixed into food such as health food as a food additive. Specific examples of products of food and drink according to the present invention which comprises the mixture of cyclic and/or straight chain poly lactic acids having a condensation degree of 3 to 19, include health foods or supplements including drinks, such as those generally called a soft drink, drinkable preparation, health food, specified supplement food, functional food, function-activating food, nutritional supplementary food, supplement, feed, feed additive and the like.

Specific examples of food and drink into which the mixture of poly lactic acids can be mixed include confectionary, such as a chewing gum, chocolate, candy, sweet tablet, jelly, cookie, biscuit, and yogurt; frozen deserts, such as ice cream and sherbet; beverages, such as tea, soft drink (including juice, coffee, cocoa and the like), nutrition supplement drinkable preparation, and cosmetic drinkable preparation; and all other food and drink, such as bread, ham, soup, jam, spaghetti, and frozen food. Alternatively, the mixture of poly lactic acids used in the present invention can also be used by adding to seasoning, food additives, and the like.

By the use of the above food and drink of the present invention, there can be provided safe food and drink which can exert the radiation protecting effect while substantially showing no toxic side effect.

The food and drink of the present invention encompasses food and drink in every form, and the types are not specifically limited. That is, the food and drink can be provided by mixing the mixture of cyclic and/or straight chain poly lactic acids having a condensation degree of 3 to 19 into the above-mentioned various food and drink, or various nutrient compositions, such as various oral or enteral nutrient preparations or drinks. Compositions of such food and drink may include protein, lipid, carbohydrate, vitamin and/or mineral, in addition to the mixture of cyclic and/or straight chain poly lactic acids having a condensation degree of 3 to 19. The form of the food and drink is not specifically limited, and may be in any form, such as solid, powdery, liquid, gel, and slurry forms, so far as it is in a form that is easily ingested.

The content of the mixture of poly lactic acids in the food and drink is not specifically limited, and is generally 0.1 to 20 weight %, more preferably approximately 0.1 to 10 weight %.

The mixture of poly lactic acids is preferably contained in the food and drink in an amount which achieve the object of the present invention. Preferably, about 0.1 g to 10 g, more preferably about 0.5 g to 3 g, of the mixture of poly lactic acids is contained per food or drink to be ingested.

The present invention is further described in the following examples, but the scope of the present invention is not limited by the examples in any way.

### EXAMPLES

### Production Example 1: Production of a mixture of poly lactic acids

500 ml of L-lactic acid (to which D-lactic acid was also mixed) was placed into a separable flask in a mantle heater. 300ml/min of nitrogen gas was flowed therein while stirring. Accumulated water was introduced into a flask equipped with a reflux condenser via a warmed descending type connecting tube, while heating at 145°C for 3 hours. Furthermore, after pressure was reduced to 150 mmHg and heated at the same temperature for 3 hours, the mixture was heated at 155°C for 3 hours under a reduced pressure of 3 mmHg, and then at 185°C for 1.5 hours under a reduced pressure of 3 mmHg to obtain poly lactic acids as a reaction product.

The obtained poly lactic acids were kept at 100°C, and 100ml of ethanol and 400ml of methanol were separately added thereto, and then the mixture was allowed to be cooled. This mixture was added to 500ml of methanol, and the mixture was well stirred and left to stand. Then, the mixture was filtrated for purification. The filtrate was subjected to vacuum drying and then dissolved in acetonitrile to obtain 200ml (stock solution) in total.

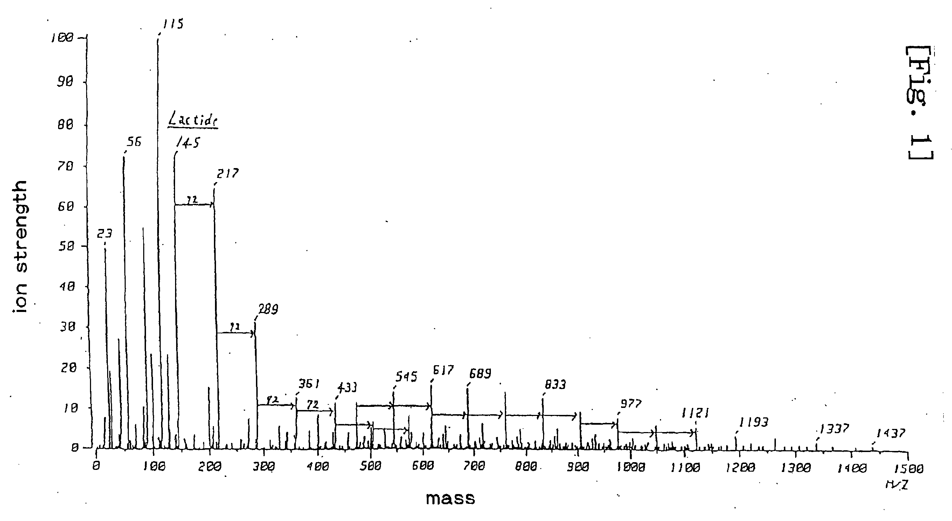

The stock solution was subjected to a reverse phase ODS column (TSK gel ODS-80 TM) which was previously equilibrated, and was stepwise eluted with 30%, 50% and 100% acetonitrile (pH2.0) each containing 0.01M hydrochloric acid to obtain poly lactic acids (condensation degree of 3 to 19) as an acetonitrile 100% elution fraction. The mass spectrum of the obtained substance is shown in Fig. 1. As is clear from the regular fragment ion peaks in Fig. 1, the obtained mixture of poly lactic acids mainly comprises cyclic condensate, and a small amount of linear condensate is contained therein.

### Test Example 1:

Eight to eleven week-old female C3H/HeLa mice were raised while kept in an acryl case. Mice were divided into a control group raised by allowing free intake of standard solid feed CE2 (obtained from Clea Japan, Inc.) and a CPL group raised by allowing free intake standard solid feed CE2 comprising 0.1% by weight (the fraction obtained by chromatography in Production Example 1 was used as is, its concentration being 0.1% by weight) of poly lactic acid mixture (hereinafter also referred to as CPL). On 55th day after initiation of CPL administration, whole-body irradiation of 4 to 8 Gy of X-rays was conducted. After irradiation, the mice were observed over the course of time and the numbers of survivingmice were counted. As a result, with irradiation of 4 to 7 Gy, all mice in the control group and the CPL group survived. However, with irradiation of 8 Gy, the mortality rate in the control group was 55.6% (5 out of 9), and the mortality rate in the CPL-administered group was 11.1% (1 out of 9).

### Test Example 2:

Mice were raised in the same manner as in Test Example 1, and on 56th day after initiation of CPL administration, whole-body irradiation with 7.5 to 9.5 Gy was performed. As a result, LD50/30 was 8.03 (7.85 to 8.20) Gy in the control group, and 13.70 (13.37 to 14.03) Gy in the CPL-administered group.

### Test Example 3:

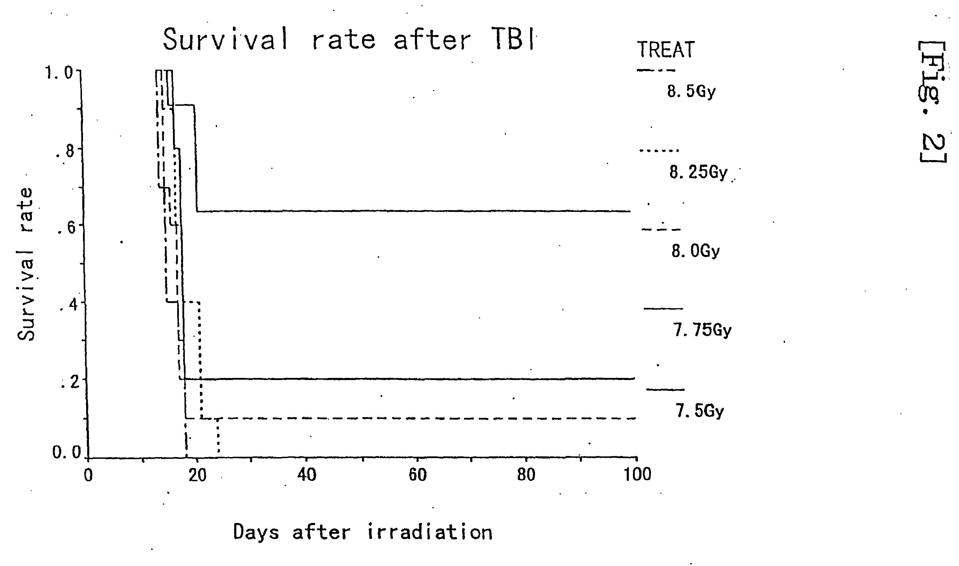

Both the control group and the CPL-administrated group (Period of CPL administration: From 13 days prior to irradiation to end of the test) were whole-body irradiated with an identical dose (7.5 to 8.5 Gy) . After irradiation, the mice were observed over the course of time and the number of surviving mice was recorded. These results are shown in Figs. 2-4.

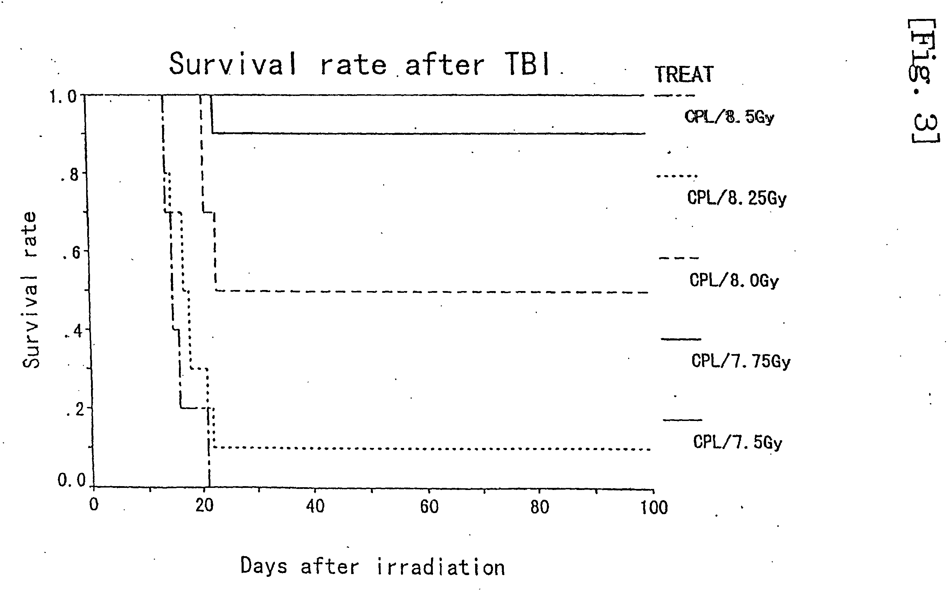

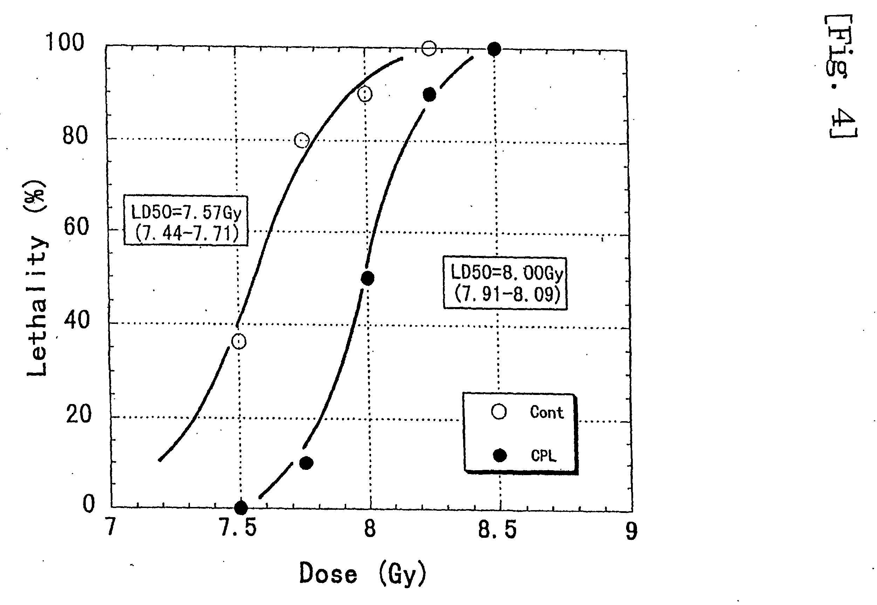

From the results of Figs. 2 and 3, it is understand that the CPL-administered group (Fig.3) has a higher survival rate than the control group (Fig. 2). Further, as indicated in Fig. 4, LD50/30 was 7.57 (7.44 to 7.71) Gy for the control group, and 8.00 (7.91 to 8.09) Gy for the CPL-administered group. From these results, it was demonstrated that, if CPL administration was initiated prior to irradiation with radiation, a radiation protection effect was exhibited.

Further, from the results of Fig. 4, it is understood that the CPL-administered group (Fig. 3) has a longer number of average surviving days than the control group (Fig. 2).

Further, the average number of surviving days on 100th day after whole-body irradiation of mice, is shown below.

| Radiation Dose | Control group | CPL group |
|---|---|---|
| 7.5 Gy | 71 days | All survived |
| 7.75 Gy | 34 days | 92 days |
| 8.0 Gy | 25 days | 61 days |
| 8.25 Gy | 19 days | 26 days |
| 8.5 Gy | 16 days | 16 days |

As is understood from these results, it was demonstrated that, if CPL administration was initiated prior to irradiation with radiation, a radiation protection effect was exhibited.

### Industrial Utility

The radiation protecting agent according to the present invention which comprises as an effective ingredient a mixture of cyclic and/or straight chain poly lactic acids having a condensation degree of 3 to 19, exhibits a radiation protecting effect against whole body irradiation with radiation when administered prior to irradiation with radiation. Further, the mixture of poly lactic acids used as an active ingredient in the present invention is a low condensate of lactic acids derived from organism components, and so it has a high biocompatibility with few side effects.

## Claims

1. A radiation protecting agent which comprises, as an active ingredient, a mixture of cyclic and/or straight chain poly lactic acids having a condensation degree of 3 to 19.

2. The radiation protecting agent of claim 1 which is used for prevention and/or therapy of disorders due to radiation therapy.

3. The radiation protecting agent of claim 1 or 2, wherein the lactic acid that is a repeating unit in the poly lactic acid consists substantially of L-lactic acid.

4. The radiation protecting agent of any one of claims 1 to 3, wherein the mixture of cyclic and/or straight chain poly lactic acids having a condensation degree of 3 to 19 is a fraction obtained by condensing lactic acids by dehydration under an inactive atmosphere, subjecting the ethanol- and methanol-soluble fractions of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

5. The radiation protecting agent of claim 4, wherein condensation by dehydration is performed by stepwise decompression and temperature rise under nitrogen gas atmosphere.

6. The radiation protecting agent of claim 4 or 5, wherein reverse phase column chromatography is performed by ODS column chromatography.

7. Food and drink for the protection against radiation , which comprises, as an active ingredient, a mixture of cyclic and/or straight chain poly lactic acids having a condensation degree of 3 to 19.
